(19) Europäisches Patentamt
European Patent Office
Office européen des brevets

(11) Veröffentlichungsnummer: **0 054 898**
**A1**

(12) # EUROPÄISCHE PATENTANMELDUNG

(21) Anmeldenummer: **81110475.1**

(22) Anmeldetag: **16.12.81**

(51) Int. Cl.³: **C 07 C 125/08**, C 07 C 143/78, A 61 K 31/275, A 61 K 31/18

(30) Priorität: **23.12.80 DE 3048613**

(43) Veröffentlichungstag der Anmeldung: **30.06.82**
**Patentblatt 82/26**

(84) Benannte Vertragsstaaten: **AT BE CH DE FR GB IT LI NL**

(71) Anmelder: **HOECHST AKTIENGESELLSCHAFT, Postfach 80 03 20, D-6230 Frankfurt/Main 80 (DE)**

(72) Erfinder: **Sinharay, Akhileswar, Dr., Landgraf-Philipp-Strasse 28, D-6000 Frankfurt am Main 50 (DE)**
Erfinder: **Granzer, Ernold, Dr. Dr., Falkensteiner Strasse 24, D-6233 Kelkheim (Taunus) (DE)**

(54) **Formamidin-Derivate, Verfahren zu ihrer Herstellung, ihre Verwendung sowie pharmazeutische Präparate auf Basis dieser Verbindungen und deren Herstellung.**

(57) Formamidin-Derivate der Formel I

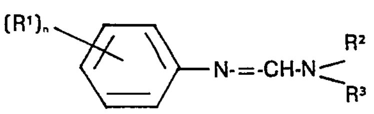

(I)

worin

$R^3$ -CN oder $-SO_2-$ 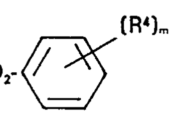

wobei
m die Zahl 1 oder 2,
$R^4$ Wasserstoff, $C_1-C_4$)-Alkyl oder Halogen bedeuten.

Verfahren zu ihrer Herstellung, pharmazeutische Präparate auf Basis dieser Verbindungen sowie ihre Verwendung zur Beeinflussung des Serumlipoproteinspiegels werden beschrieben.

EP 0 054 898 A1

0054898

HOECHST AKTIENGESELLSCHAFT HOE 80/F 294 Dr.D/Pa

# Formamidin-Derivate, Verfahren zu ihrer Herstellung, ihre Verwendung sowie pharmazeutische Präparate auf Basis dieser Verbindungen und deren Herstellung.

Die Erfindung betrifft Formamidin-Derivate der allgemeinen Formel I

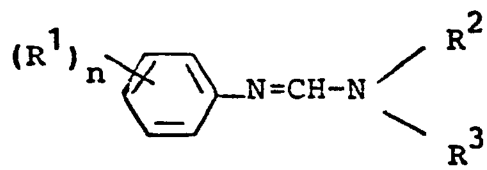

(I)

die wertvolle pharmakologische Eigenschaften besitzen und daher als Arzneimittel verwendet werden können.
In der Formel bedeuten:

n o oder eine Zahl von 1 bis 3

$R^1$ Halogen, $(C_1 - C_4)$-Alkyl, $(C_1 - C_4)$-Alkoxy, $(C_1 - C_4)$-Alkylthio, Trifluormethyl, Nitro, Cyano, Phenoxy, Mono- oder Dihalophenoxy, Phenylthio oder Mono- oder Dihalogenphenylthio, wobei für n = 2 oder 3 die Reste $R^1$ gleich oder verschieden sein können,

$R^2$ $(C_1 - C_6)$-Alkyl, $(C_1 - C_4)$-Alkoxy -$(C_1 - C_4)$-alkyl, Phenoxy-$(C_1 - C_4)$-alkyl, Cyano-$(C_1 - C_4)$-alkyl, $(C_1 - C_4)$-Alkoxycarbonyl-$(C_1 - C_4)$-alkyl, Benzyl, Allyl, Propargyl, ein Alkylacetal der Formel $(R^5O)_2CH$- oder $(R^5O)_2-R^6$, worin $R^5$ und $R^6$ gleiche oder verschiedene Alkylreste mit 1 bis 4 C-Atomen bedeuten, oder $R^2$ bedeutet einen Rest der Formel $(C_1 - C_4)$-Alkyl- 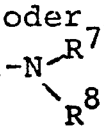 , worin $R^7$ und $R^8$ jeweils gleiche oder verschiedene $(C_1 - C_4)$-Alkyl bedeuten oder zusammen mit dem N-Atom einen heterocyclischen Ring mit bis zu 6 C-Atomen bilden, wobei 1 C-Atom durch ein O- oder N-Atom ersetzt sein kann und wobei dieses N-Atom mit $(C_1 - C_4)$-Alkyl substituiert sein kann,

$R^3$ -CN oder $-SO_2-$ 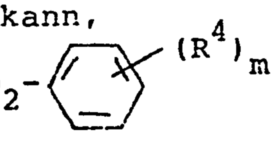

wobei

m die Zahl 1 oder 2,

$R^4$ Wasserstoff, $(C_1 - C_4)$-Alkyl oder Halogen bedeutet.

Der Substituent $R^1$ bedeutet vorzugsweise Chlor, Methyl, Trifluormethyl, Phenoxy, Phenylthio und n ist vorzugsweise 1 oder 2.

Falls im Substituenten $R^2$ eine $(C_1 - C_4)$-Alkyl- oder $(C_1 - C_4)$-Alkoxygruppe vorkommt, so sind solche Gruppen bevorzugt, die ein bis zwei C-Atome besitzen. $R^2$ bedeutet vorzugsweise geradkettiges oder verzweigtes $(C_1 - C_4)$-Alkyl und $R^3$ vorzugsweise -CN oder die p-Toluolsulfonylgruppe. Ganz besonders bevorzugt sind Verbindungen, worin n = 1 ist und $R^1$ Phenoxy oder Phenylthio oder worin n = 2 ist und $R^1$ jeweils Chlor darstellt. Als Substituent $R^2$ kommen insbesondere der Methyl- und Butylrest in Betracht.

Die Erfindung betrifft weiter ein Verfahren zur Herstellung der Verbindungen der Formel I, das dadurch gekennzeichnet ist, daß man ein Formamidin-Derivat der allgemeinen Formel II,

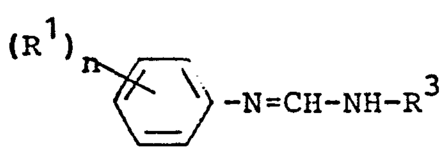

(II)

mit einer Verbindung der allgemeinen Formel III

$$R^2 - X \qquad \text{(III)}$$

worin X eine Abgangsgruppe bedeutet, umsetzt.

Die Ausgangsstoffe der allgemeinen Formel II,worin $R^3$-CN bedeutet, sind entweder in der Literatur beschrieben worden oder können nach den dort beschriebenen Methoden hergestellt werden(z.B. H. Schäfer und K. Gewald, Journal f. prakt. Chemie 318, 347 (1976)).

0054898

Die Ausgangsstoffe der allgemeinen Formel II, worin $R^3$

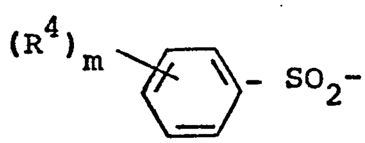

bedeutet, sind ebenfalls in der Literatur beschrieben worden oder können in analoger Weise wie dort angegeben aus Arylsulfonamiden und Arylisocyanaten in Gegenwart von CuCl als Katalysator (P. Jakobsen und S. Treppendahl, Tetrahedron 33, 3137 (1977)) oder auch durch die Umsetzung von entsprechenden N-Phenylsulfonyl-formimidsäurealkylester mit den entsprchenden Arylaminen (US-Patent 3,953,492 und 3,953,493) hergestellt werden.

Die Ausgangsstoffe der allgemeinen Formel II, worin $R^3$

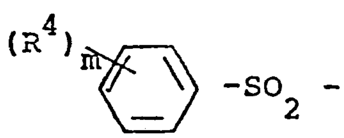

bedeutet, sind ferner nach einem bisher noch nicht beschriebenen Eintopfverfahren in höherer Ausbeute zugänglich, wobei die entsprechenden Arylsulfonamide und Arylamine mit Orthoameisensäure-trialkylester unter Rückfluß erhitzt werden. Die Umsetzung erfolgt nach folgendem Schema:

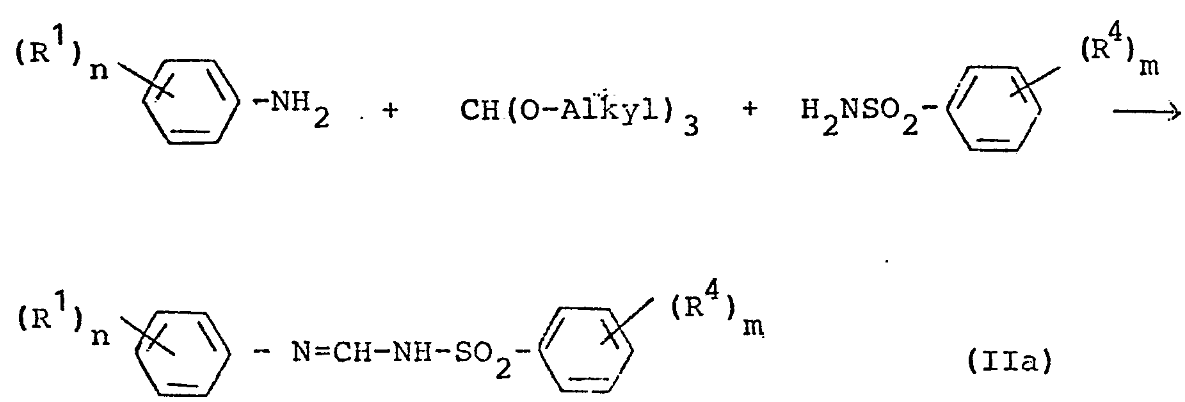

(IIa)

Beim Abkühlen fallen die erwünschten Produkte der Formel IIa aus, die nach Absaugen aus einem geeigneten Lösungsmittel umkristallisiert werden.

Die Verbindungen der allgemeinen Formel I werden dadurch hergestellt, daß man $N^1$-Cyano- bzw. $N^1$-Sulfonyl-formamidine der allgemeinen Formel II mit einer Verbindung der allgemeinen Formel III umsetzt. Als Abgangsgruppe X eignen sich z.B. Halogen, vorzugsweise Jod, Brom oder Chlor, oder eine Arylsulfonsäureestergruppe, vorzugsweise eine Benzolsulfonsäure- oder Toluolsulfonsäureestergruppe.

Die Umsetzungen werden zweckmäßig mit äquimolaren Mengen der jeweiligen Ausgangsstoffe durchgeführt, vorteilhaft in einem polaren Lösungsmittel, z.B. Alkoholen wie Methanol, Äthanol, Propanol, Methoxyäthanol, Ketonen wie Aceton, Äthylmethylketon oder Amiden wie Dimethylformamid, Dimethylacetamid, N-Methylpyrrolidon, Tetramethylharnstoff, Hexamethylphosphorsäureamid oder in Dimethylsulfoxid.

Im Falle der Bedeutung von X in Formel III = Halogen empfiehlt sich die Anwendung eines säurebindenden Mittels. Als solche kommen Basen wie Triäthylamin oder Pyridin, sowie Alkali- und Erdalkalicarbonate und -bicarbonate, -hydroxide und -alkoxide, wie z.B. -methoxide, -ethoxide, -butoxide in Frage.

Die Reaktionstemperaturen können im allgemeinen zwischen $0^{o}$ und $150^{o}$ liegen, vorzugsweise zwischen $20^{o}$ und $80^{o}$C. Wenn man stark polares aprotisches Lösungsmittel wie z.B. Dimethylformamid, Dimethylsulfoxid verwendet, wird bevorzugt bei Raumtemperatur gearbeitet.

Die Reaktionszeiten betragen, je nach Temperaturbereich und Lösungsmittel, einige Minuten bis mehrere Stunden.

Die Isolierung der Verfahrensprodukte erfolgt nach üblichen Methoden z.B. durch Abdestillieren der verwendeten Lösungsmittel oder Verdünnen der Reaktionslösung mit Wasser. Die Reinigung kann durch Umkristallisieren aus einem geeigneten Lösungsmittel oder Lösungsmittelgemisch erfolgen. Geeignet sind beispielsweise niedermolekulare Alkohole.

Die erfindungsgemäßen Verbindungen der Formel I sind wertvolle Arzneimittel und zeichnen sich durch eine sehr günstige Wirkung auf die Serumlipoproteine aus. Sie können daher als Arzneimittel, insbesonders zur Beeinflussung der Serumlipoproteine, verwendet werden. Die Erfindung betrifft daher auch pharmazeutische Präparate auf Basis der Verbindungen der Formel I sowie die Verwendung als Arzneimittel.

Formamidine mit günstiger Wirkung auf den Lipidspiegel sind bisher nicht bekannt. Es war nun überraschend, daß die erfindungsgemäßen Verbindungen der Formel I eine sehr starke und günstige Beeinflussung der Serumlipoproteine zeigen.

Es ist allgemein anerkannt, daß für die Entstehung arteriosklerotischer Gefäßveränderungen, insbesondere der coronaren Herzkrankheit, Hyperlipoproteinämien einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Hierbei kommt es aber auf ganz bestimmte Klassen von Serum-Lipoproteinen an, da die low density (LDL) und very low density-Lipoproteine (VLDL) einen atherogenen Risikofaktor darstellen, während die high density-Lipoproteine (HDL) eine Schutzfunktion gegenüber der coronaren Herzkrankheit darstellen. Hypolipidämika sollen demnach VLDL-Cholesterin und LDL-Cholesterin im Serum erniedrigen, dabei aber die HDL-Cholesterin-Konzentration nach Möglichkeit unbeeinflußt lassen oder sogar erhöhen. Die erfindungsgemäßen Verbindungen haben wertvolle therapeutische Eigenschaften. So erniedrigen sie vor allem die Konzentration von LDL und VLDL, während die HDL-Fraktion entweder in wesentlich geringerem Maße erniedrigt, oder sogar erhöht wird. Sie können daher zur

Prophylaxe und Regression von atherosklerotischen Veränderungen herangezogen werden, indem sie einen kausalen Risikofaktor ausschalten. Hierzu zählen nicht nur die primären Hyperlipoproteinämien, sondern auch gewisse sekundäre Hyperlipidämien wie sie z.B. beim Diabtes vorkommen. Das relative Lebergewicht wird durch die Verbindungen I in wirksamer Dosis nicht verändert, während das als hypolipidämischer Standard verwendete Clofibrat zu einer starken Erhöhung des relativen Lebergewichts führt.

Die Wirkung der angeführten Verbindungen auf die Serum-Lipoproteine wurde an männlichen Wistar-Ratten untersucht, die 7 Tage per Schlundsonde mit den angeführten in Polyäthylenglykol 400 suspendierten Verbindungen behandelt wurden. Außerdem wurde eine Kontrollgruppe, die nur das Lösungsmittel Polyäthylenglykol 400 erhielt, mitgeführt, sowie eine Ratten-Gruppe, die zum Vergleich das Standardhypolipidämikum Clofibrat, ebenfalls in Polyäthylenglykol 400 gelöst, erhielt. Pro Gruppe wurden in der Regel 10 Tiere eingesetzt, denen am Ende der Behandlung das Blut nach leichter Äthernakose aus dem Orbitalplexus entnommen wurde. Das daraus gewonnene Serum wurde gepoolt. Die Serum-Lipoproteine wurden in der Ultrazentrifuge in folgende Dichteklassen getrennt:
VLDL 1,006; LDL 1,006 bis 1,04; HDL 1,04 bis 1,21.

Aus den in der Ultrazentrifuge isolierten Lipoprotein-Fraktionen wurde das darin enthaltene Choletserin vollenzymatisch nach der CHOD-PAP-Methode mittels der Testkombination von Boehringer Mannheim bestimmt und die Werte in µg/ml Serum umgerechnet. In der angeführten Tabelle (S.8) ist die Veränderung des Lipoprotein-Cholesterins in der behandelten Gruppe gegenüber einer unter gleichen Bedingungen mitgeführten Kontrollgruppe angegeben. Wie aus der Tabelle ersichtlich, bewirkt Clofibrat eine etwa gleichstarke Senkung der LDL-Fraktion und der HDL-Fraktion, während die neuen Verbindungen eine starke selektiv senkende

Wirkung auf die atherogenen Lipoproteinfraktionen (VLDL und LDL) ausüben und die schützende HDL-Fraktion im wesentlichen unbeeinflußt lassen oder sogar vermehren.

Als therapeutische Zubereitung der Verbindungen der Formel I kommen vor allem Tabletten, Dragees, Kapseln, Supposotorien und Säfte in Frage. Die neuen Verbindungen können dabei entweder allein oder mit pharmakolisch annehmbaren Trägern vermischt, angewandt werden. Eine orale Anwendungsform wird bevorzugt. Zu diesem Zweck werden die aktiven Verbindungen vorzugsweise mit an sich bekannten Substanzen vermischt und durch an sich bekannte Methoden in geeignete Darreichungsformen gebracht, wie Tabletten, Steckkapseln, wäßrige oder ölige Suspensionen oder wäßrige oder ölige Lösungen. Als inerte Träger können z.B. Magnesiumcarbonat, Milchzucker oder Maisstärke unter Zusatz anderer Stoffe wie z.B. Magnesiumstearat verwendet werden. Dabei kann die Zubereitung als Trocken- oder Feuchtgranulat erfolgen. Als ölige Trägerstoffe oder Lösungsmittel kommen besonders pflanzliche und tierische Öle in Betracht wie z.B. Sonnenblumenöl oder Lebertran. Als tägliche Dosis kommen etwa 50 mg bis 5 g, vorzugsweise 250 bis 1000 mg, in Betracht. Eine Dosierungseinheit enthält 50 bis 1000 mg, vorzugsweise 250 bis 500 mg.

Die Zubereitungen können bei der Behandlung von Lipidstoffwechselstörungen außer den üblichen Füll- und Trägerstoffen noch einen weiteren Wirkstoff enthalten wie z.B. ein Antihypertensivum, wie beispielsweise ein Saluretikum, Reserpin, Hydralazin, Guanethidin, α-Methyldopa, Clonidin oder ein β-Sympathikolytikum oder eine antihyperurikämisch wirksame Verbindung, ein orales Antidiabetikum, ein Geriatrikum oder eine Verbindung mit durchblutungssteigernder Wirkung.

% Veränderung der Lipoproteine in Ratten-Serum nach siebentägiger oraler Behandlung mit den Verbindungen

| Beispiel | Dosis mg/kg/Tag | % Veränderung des Cholesterins gegenüber Kontrolle: im Serum | in der Serum-Lipoprotein-Fraktion: VLDL | LDL | HDL |
|---|---|---|---|---|---|
| 2 | 10 | -4 | -50 | -22 | +3 |
| 3 | 10 | -5 | -32 | -22 | +8 |
| 4 | 10 | -17 | -67 | -33 | -7 |
| | 3 | - 5 | -23 | - 8 | +2 |
| 6 | 10 | - 8 | -67 | -25 | -1 |
| | 3 | - 4 | -23 | -22 | +3 |
| 7 | 10 | - 1 | -51 | -22 | +5 |
| 12 | 10 | - 6 | -70 | -42 | -1 |
| 13 | 10 | - 6 | -100 | -39 | +1 |
| 25 | 10 | - 5 | - 70 | -45 | +5 |
| 27 | 3 | -18 | - 13 | -50 | -2 |
| 28 | 3 | - 5 | - 5 | -25 | +2 |
| 30 | 10 | - 3 | ±0 | -27 | -1 |
| 34 | 10 | +10 | - | -36 | +14 |
| 35 | 3 | + 1 | -52 | -37 | -2 |
| | 1 | + 7 | -72 | -19 | +2 |
| | 10 | + 4 | - | -36 | +2 |
| 36 | 10 | - 2 | - | -34 | +3 |
| | 3 | ± 0 | ±0 | -19 | +1 |
| 37 | 10 | - 8 | ±0 | -51 | +3 |
| 38 | 10 | - 8 | -30 | -36 | +2 |
| 39 | 3 | - 3 | -26 | -26 | -7 |
| 48 | 3 | - 8 | ±0 | -56 | -2 |
| 49 | 3 | + 2 | ±0 | -48 | +2 |
| 50 | 3 | + 7 | ±0 | -35 | +3 |
| | 1 | +13 | +3 | -21 | +10 |
| Clofibrat | 100 | -24 | -30 | -25 | -33 |

Die nachfolgenden Beispiele sollen die Erfindung erläutern.

Beispiel 1:

$N^1$-Äthyl-$N^1$-p-toluolsulfonyl-$N^2$-(4-phenoxyphenyl)-formamidin

9,15 g $N^2$-(4-Phenoxyphenyl)-$N^1$-p-toluolsulfonylformamidin werden in 50 ml Dimethylformamid gelöst, mit 3,45 g Kaliumcarbonat versetzt, unter Rühren bei 20 - 25°C 3,9 g Äthyljodid zugetropft und anschließend 7 Stunden bei Raumtemperatur gerührt. Das Reaktionsgemisch wird dann in 300 ml Eiswasser gegeben, 16 Stunden stehengelassen und das ausgefallene Produkt abgesaugt und aus Äthanol umkristallisiert. Ausbeute: 7,9 g
Schmp. 113 - 115°C

Das als Ausgangsmaterial verwendete $N^2$-(4-Phenoxy-phenyl)-$N^1$-p-toluolsulfonylformamidin wird in folgender Weise hergestellt:

92,5 g p-Phenoxy-anilin, 81,5 g Orthoameisensäuretriäthylester und 85,5 g p-Toluol-sulfonamid werden 30 Minuten unter Rückfluß gekocht, auf Raumtemperatur abgekühlt, das ausgefallene Produkt abgesaugt und aus Methylglykol umkristallisiert. Ausbeute: 104 g,
Schmp. 174 - 176°C.

In analoger Weise erhält man unter Verwendung der entsprechenden Ausgangsstoffe, die in der nachstehenden Tabelle 1 zusammengefaßten Verbindungen der Formel I:

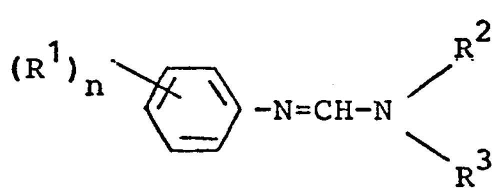

(I)

Tabelle 1

| Beispiel | $(R^1)n$ | $R^2$ | $R^3$ | Schmp. (°C) |
|---|---|---|---|---|
| 2 | 4-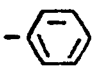-O- | $C_6H_5CH_2-$ | $CH_3-$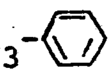$-SO_2-$ | 152-153 |
| 3 | " | $C_3H_7-$ | " | 98-100 |
| 4 | " | $(CH_3)_2CH-$ | " | 125-130 |
| 5 | " | $C_4H_9-$ | " | 75- 77 |
| 6 | " | $CH_2=CHCH_2-$ | " | 97- 98 |
| 7 | " | $CH{\equiv}CCH_2-$ | " | 100-101 |
| 8 | " | $NC-CH_2-$ | " | 135-137 |
| 9 | " | $(C_2H_5O)_2CHCH_2$ | " | 88- 89 |
| 10 | " | $C_6H_5OCH_2CH_2-$ | " | 146-147 |
| 11 | " | $C_2H_5O_2C-CH_2-$ | " | 79- 80 |
| 12 | " | $CH_3-$ | " | 120-121 |
| 13 | 4-Cl 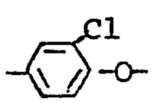 | $CH_3$ | " | 154-155 |
| 14 | " | $C_4H_9-$ | " | 75- 76 |
| 15 | " | $CH_2=CHCH_2-$ | " | 101-102 |
| 16 | " | $C_2H_5O_2CCH_2-$ | " | 105-106 |
| 17 | " | $NC-CH_2-$ | " | 130-131 |
| 18 | 4-Cl 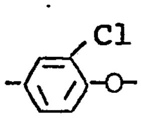 | $CH{\equiv}CCH_2-$ | " | 106-107 |
| 19 | " | $(CH_3)_2CH-$ | " | 114-116 |
| 20 | " | $C_3H_7-$ | " | 103-104 |
| 21 | " | $C_2H_5-$ | " | 117-118 |

Tabelle 1 (Fortsetzung)

| Beispiel | $(R^1)n$ | $R^2$ | $R^3$ | Schmp. (°C) |
|---|---|---|---|---|
| 22 | 4-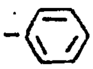-S- | NC-$CH_2$- | $CH_3$-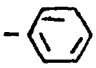-$SO_2$- | 119-120 |
| 23 | " | $CH_2$=CH$CH_2$- | " | 112-113 |
| 24 | " | CH≡C$CH_2$- | " | 133-134 |
| 25 | " | $CH_3$- | " | 114-115 |
| 26 | " | $C_6H_5CH_2$- | " | 108-109 |
| 27 | " | $C_4H_9$- | " | 93- 95 |
| 28 | " | $(CH_3)_2$CH- | " | 102-108 |
| 29 | " | $C_3H_7$- | " | 112-113 |
| 30 | " | $C_2H_5$- | " | 122-123 |
| 31 | " | 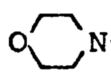-$CH_2CH_2$- | " | 119-120 |
| 32 | " | $(C_2H_5)_2NCH_2CH_2$- | " | 121-122 |
| 33 | 2-CH$(CH_3)_2$ | $CH_3$- | " | 133-134 |
| 34 | 2,4,6-$(CH_3)_3$ | $CH_3$- | " | 166 |
| 35 | 2,4-$Cl_2$ | " | " | 136 |
| 36 | 3,5-$(CF_3)_2$ | " | " | 158-162 |
| 37 | 2-$CH_3$ | " | " | 129-130 |
| 38 | 4-Cl,2-$CH_3$ | " | " | 148-149 |
| 39 | 4-Cl,2-$CF_3$ | " | " | 141-143 |
| 40 | 2,4-$(CH_3)_2$ | " | " | 135-136 |
| 41 | H | " | -CN | 87- 88 |
| 42 | 2,4-$Cl_2$ | " | -CN | 120-121 |

| Beispiel | $(R^1)n$ | $R^2$ | $R^3$ | Schmp. (°C) |
|---|---|---|---|---|
| 43 | 4-Cl,2-$CH_3$ | $CH_3$- | -CN | 119 |
| 44 | 3,5-$(CF_3)_2$ | " | " | 128 |
| 45 | 2,4-$(CH_3)_2$ | " | " | 71- 73 |
| 46 | 2,4,6-$(CH_3)_3$ | " | " | 108-109 |
| 47 | 2,6-$(CH_3)_2$ | " | " | 150-152 |
| 48 | 4- 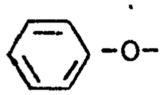 | " | " | 90- 94 |
| 49 | 4- 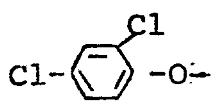 | " | " | 150-152 |
| 50 | 3- Cl, 4- 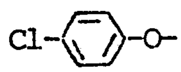 | " | " | 109-110 |
| 51 | 4- 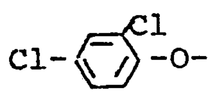 | $C_2H_5$- | " | 97- 99 |
| 52 | 4-Cl | $CH_3$- | 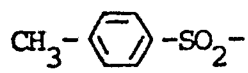 | 148-150 |

Patentansprüche:

1. Formamidin-Derivate der allgemeinen Formel I

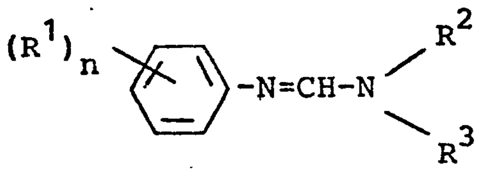

(I)

worin n o oder eine Zahl von 1 bis 3,

$R^1$ Halogen, $(C_1 - C_4)$-Alkyl, $(C_1 - C_4)$-Alkoxy, $(C_1 - C_4)$- Alkylthio, Trifluormethyl, Nitro, Cyano, Phenoxy, Mono- oder Dihalophenoxy, Phenylthio oder Mono- oder Dihalogenphenylthio, wobei für n = 2 oder 3 die Reste $R^1$ gleich oder verschieden sein können,

$R^2$ $(C_1 - C_6)$-Alkyl, $(C_1 - C_4)$-Alkoxy-$(C_1 - C_4)$-alkyl, Phenoxy-$(C_1 - C_4)$-alkyl, Cyano-$(C_1 - C_4)$-alkyl, $(C_1 - C_4)$-Alkoxycarbonyl-$(C_1 - C_4)$-alkyl, Benzyl, Allyl, Propargyl, ein Alkylacetal der Formel $(R^5O)_2CH$- oder $(R^5O)_2$-$R^6$, worin $R^5$ und $R^6$ gleiche oder verschiedene Alkylreste mit 1 bis 4 C-Atomen bedeuten, oder $R^2$ bedeutet einen Rest der Formel $(C_1 - C_4)$ -

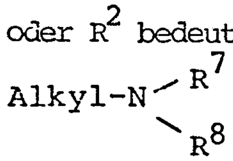

, worin $R^7$ und $R^8$ jeweils gleiche oder verschiedene $(C_1 - C_4)$-Alkylreste bedeuten oder zusammen mit dem N-Atom einen heterocyclischen Ring mit bis zu 6 C-Atomen bilden, wobei 1 C-Atom durch ein O- oder N-Atom ersetzt sein kann und wobei dieses N-Atom mit $(C_1 - C_4)$-Alkyl substituiert sein kann ,

$R^3$ -CN oder

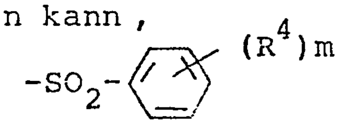

wobei

m die Zahl 1 oder 2,

$R^4$ Wasserstoff, $(C_1 - C_4)$-Alkyl oder Halogen

bedeuten.

2. Verfahren zur Herstellung der Verbindungen der Formel I, das dadurch gekennzeichnet ist, daß man ein Formamidin-Derivat der allgemeinen Formel II,

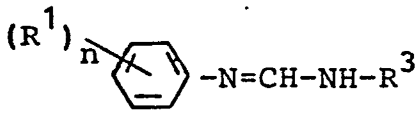 (II)

mit einer Substanz der allgemeinen Formel III,

$$R^2 - X \qquad \text{(III)}$$

worin X eine Abgangsgruppe bedeutet, umsetzt.

3. Pharmazeutische Präparate auf Basis von Verbindungen gemäß Anspruch 1.

4. Pharmazeutische Präparate mit das Serumlipoproteinspektrum beeinflußender Wirkung, bestehend aus bzw. enthaltend eine Verbindung gemäß Anspruch 1.

5. Verfahren zur Herstellung pharmazeutischer Präparate gemäß Ansprüchen 3 und 4, dadurch gekennzeichnet, daß man eine Verbindung gemäß Anspruch 1 gegebenenfalls mit pharmazeutischen Trägern und/oder Stabilisatoren in eine für therapeutische Zwecke geeignete Anwendungsform bringt.

6. Verfahren zur Behandlung von Störungen des Serumlipotroeinspektrums, dadurch gekennzeichnet, daß man eine wirksame Menge einer Verbindung gemäß Anspruch 1 verabreicht.

7. Verbindung gemäß Anspruch 1 der Formel

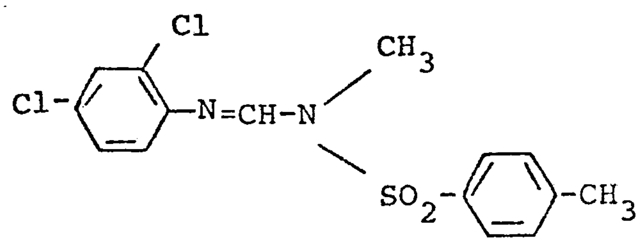

8. Verbindung gemäß Anspruch 1 der Formel

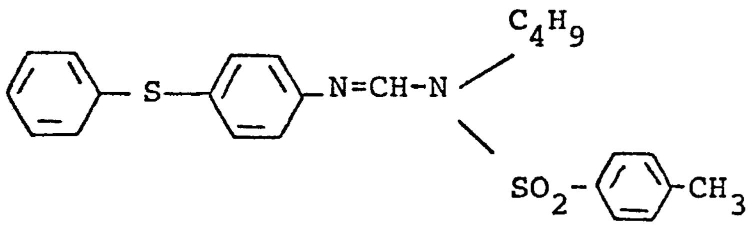

9. Verbindung gemäß Anspruch 1 der Formel

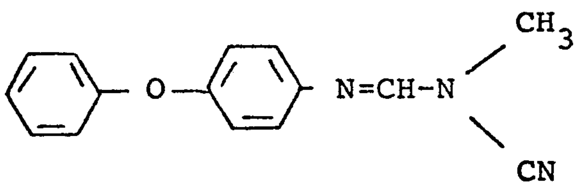

HOE 80/F 294
0054898

Patentansprüche für Österreich:

1. Verfahren zur Herstellung von Formamidin-Derivaten der allgemeinen Formel I

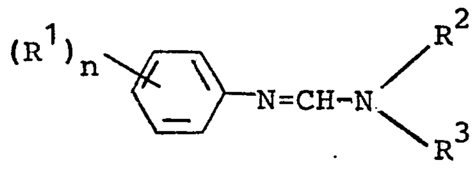

(I)

worin n o oder eine Zahl von 1 bis 3,

$R^1$ Halogen, $(C_1 - C_4)$-Alkyl, $(C_1 - C_4)$-Alkoxy, $(C_1 - C_4)$-Alkylthio, Trifluormethyl, Nitro, Cyano, Phenoxy, Mono- oder Dihalophenoxy, Phenylthio oder Mono- oder Dihalogenphenylthio, wobei für n = 2 oder 3 die Reste $R^1$ gleich oder verschieden sein können,

$R^2$ $(C_1 - C_6)$-Alkyl, $(C_1 - C_4)$-Alkoxy-$(C_1 - C_4)$-alkyl, Phenoxy-$(C_1-C_4)$-Alkyl, Cyano-$(C_1 - C_4)$-alkyl, $(C_1 - C_4)$-Alkoxycarbonyl-$(C_1 - C_4)$-alkyl, Benzyl, Allyl, Propargyl, ein Alkylacetal der Formel $(R^5O)_2CH-$ oder $(R^5O)_2-R^6$, worin $R^5$ und $R^6$ gleiche oder verschiedene Alkylreste mit 1 bis 4 C-Atomen bedeuten, oder $R^2$ bedeutet einen Rest der Formel $(C_1 - C_4)$ -

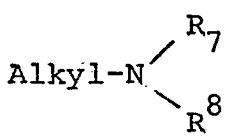

, worin $R^7$ und $R^8$ jeweils gleiche oder verschiedene $(C_1 - C_4)$-Alkylreste bedeuten oder zusammen mit dem N-Atom einen heterocyclischen Ring mit bis zu 6 C-Atomen bilden, wobei 1 C-Atom durch ein O- oder N-Atom ersetzt sein kann und wobei dieses N-Atom mit $(C_1 - C_4)$-Alkyl substituiert sein kann,

$R^3$ -CN oder 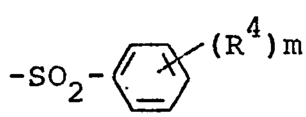

wobei

0054898

m die Zahl 1 oder 2,

$R^4$ Wasserstoff, $(C_1 - C_4)$-Alkyl oder Halogen

bedeuten, dadurch gekennzeichnet, daß man ein Formamidin-Derivat der allgemeinen Formel II,

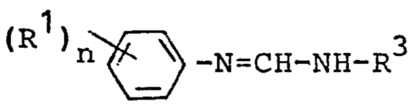

(II)

mit einer Substanz der allgemeinen Formel III,

$$R^2 - X \qquad \text{(III)},$$

worin X eine Abgangsgruppe bedeutet, umsetzt.

2.) Verfahren gemäß Anspruch 1, dadurch gekennzeichnet, daß man die Verbindung der Formel

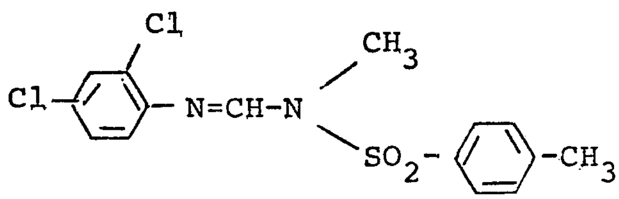

herstellt.

3.) Verfahren gemäß Anspruch 1, dadurch gekennzeichnet, daß man die Verbindung der Formel

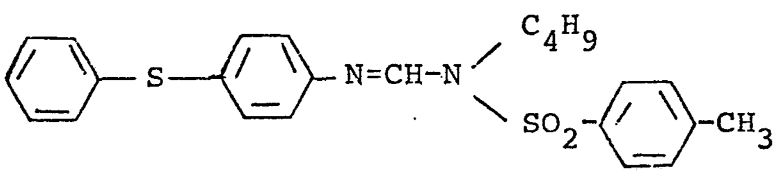

herstellt.

4.) Verfahren gemäß Anspruch 1, dadurch gekennzeichnet, daß man die Verbindung der Formel

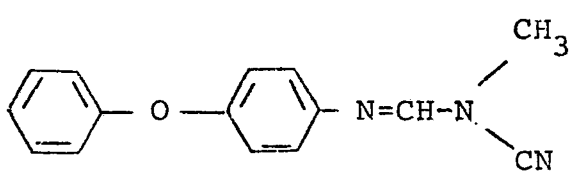

herstellt.

Europäisches Patentamt

# EUROPÄISCHER RECHERCHENBERICHT

0054898

Nummer der Anmeldung

EP 81 11 0475

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.) |
|---|---|---|---|
| X | US - A - 3 962 305 (PALLOS)<br>* Ansprüche; Beispielen *<br>-- | 1-2 | C 07 C 125/08<br>C 07 C 143/78<br>A 61 K 31/275<br>A 61 K 31/18 |
| X | TETRAHEDRON, Band 33, 1977 Pergamon Press OXFORD (GB) P. JAKOBSEN et al.:"N-Sulphonylformamidines; Preparation and Characterisation", Seiten 3137-3140<br>* Seite 3138 *<br>-- | 1-2 | RECHERCHIERTE SACHGEBIETE (Int Cl.)<br>C 07 C 125/00<br>C 07 C 143/00 |
| X | CHEMISCHE BERICHTE, Band 94, No. 10, 1961 WEINHEIM (DE) TOSOLINI: "Uber IR-Spektren N'. N'- disubstituierter N-Benzolsulfonyl- und N-Methansulfonyl-formamidine", Seiten 2731-2732 *<br>* Tabelle, Seite 2732 *<br>-- | 1-2 | |
| X | TETRAHEDRON, Band 28, Nr. 18, September, 1972 Pergamon Press OXFORD (GB) A. KREUTZBERGER: "Cyanamid als NH-Aktive Komponente in der Synthese von Dehydro-N-Mannich-Basen", Seiten 4877-4880<br>* Seite 4878 *<br>------- | 1-2 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 01-04-1982 | GAUTIER |